# EUROPEAN PATENT SPECIFICATION

(11) **EP 4 100 086 B1**
(45) Date of publication and mention of the grant of the patent: **10.01.2024**
(21) Application number: 21708447.4
(22) Date of filing: 29.01.2021
(51) Int. Cl.: A61M 11/00, A61M 11/02, A61M 11/06, A61M 16/06, A61M 16/14

(54) **DRUG DELIVERY HUB FOR VENTILATION MASK**
MEDIKAMENTENVERABREICHUNGSSTELLE FÜR BEATMUNGSMASKE
MOYEU D'ADMINISTRATION DE MÉDICAMENT POUR MASQUE DE VENTILATION

(30) Priority: 06.02.2020 US 202062971088 P
(43) Date of publication of application: 14.12.2022
(73) Proprietor: SunMed Group Holdings, LLC, Grand Rapids, MI 49544 (US)
(72) Inventor: VARGA, Christopher M., Laguna Hills, California 92653 (US); REDFORD, Ryan G., Irvine, California 92618 (US); DILLINGHAM, Thomas, Aliso Viejo, California 92656 (US); PIERRO, Brian W., Yorba Linda, California 92886 (US)
(74) Representative: Epping - Hermann - Fischer
(86) International application number: PCT/US2021/015868
(87) International publication number: WO 2021/158447

(56) References cited:
- EP-A1- 2 679 264
- US-A- 4 588 129
- US-A1- 2007 283 951
- US-A1- 2019 255 274

## Description

### FIELD OF THE INVENTION

The present disclosure generally relates to nebulizer devices, and, in particular, to nebulizer devices configured to couple with a ventilation mask.

### BACKGROUND

Supplemental gas (e.g., air or oxygen) delivery to patients is a well-known treatment for a number of illnesses and conditions. For patients with respiratory difficulties, oxygen may be provided from an oxygen supply (e.g., pressurized cylinder, etc.) through a ventilation mask. During respiratory therapy or support with a ventilation mask, it is often desirable to also treat a patient with therapeutic drugs or medicaments.

In some applications, during supplemental gas therapy administered via a ventilation mask, administration of a therapeutic medicament can interrupt or diminish the effectiveness of the supplemental gas therapy. Interrupting the supplemental gas therapy to administer a medicament can also diminish the effectiveness of the medicament itself.

EP 2 679 264 A1 discloses a gas mist inhaler, in which a gas introduction part of heightening pressure for supplying a gas mist is made integral with a gas mist generator in order to realize a simplified structure and cost reduction. This gas mist inhaler is provided with a gas supply means, a gas mist generation means and an inhalation member, and the gas mist generation means forms integrally a connection part to the gas supply means, a diverging part of branching a gas flow from the connection part, a liquid storage, a nozzle of discharging one-side gas flow branched by the diverging part, a liquid suction pipe of sending the liquid to a front end of the nozzle, a collision member of colliding the liquid blown up by the gas flow discharged from the nozzle with this collision member, a confluent part of joining the generated gas mist with the gas from an upward side, a gas introduction part of leading the other-side gas flow branched by the diverging part until the confluent part, and a gas mist discharge part of collecting and discharging the generated gas mist and gas.

US 2007/283951 A1 refers to A respiratory therapy mask assembly which comprises a mask configured to be secured on a patient's face, a substantially hollow pipe having an upper end secured in the mask inlet port, a lower end configured to receive a gas inlet pipe adapter, and an angled side pipe extension configured to receive a valved nebulizer adapter assembly, a gas inlet pipe adapter and integral gas inlet pipe secured in the lower end of the hollow pipe, and a valved nebulizer adapter assembly secured in the angled pipe extension and having a reciprocally movable spring-loaded valve biased to close, and a valve actuator configured to cooperate with a nebulizer for opening the spring-loaded valve.

### SUMMARY

The invention is defined by the claims. In the following, parts of the description and drawings referring to embodiments which are not covered by the claims are not presented as embodiments of the invention, but as examples useful for understanding the invention.

The disclosed subject matter relates to drug delivery hubs. In certain embodiments, a drug delivery hub is disclosed that comprises a hub housing defining a housing volume, wherein the hub housing is configured to directly couple with a mask defining a patient cavity; a reservoir disposed at least partially within the housing volume, wherein the reservoir comprises a reservoir volume configured to dispense a medicament; a nebulizer channel in fluid communication with the reservoir volume and configured to be in fluid communication with the patient cavity when the hub is coupled with the mask; and a gas channel in fluid communication with the nebulizer channel, wherein the gas channel is configured to direct a gas flow toward the patient cavity through the nebulizer channel, drawing a portion of the medicament from the reservoir through the nebulizer channel with the gas flow toward the patient cavity.

In certain embodiments, a ventilation mask is disclosed that comprises a mask body defining a patient cavity, the mask body comprising a patient opening in fluid communication with the patient cavity; and a drug delivery hub, comprising: a hub housing defining a housing volume, wherein the hub housing is directly coupled to the mask body; a reservoir disposed at least partially within the housing volume, wherein the reservoir comprises a reservoir volume configured to dispense a medicament; a nebulizer channel in fluid communication with the reservoir volume and configured to be in fluid communication with the patient cavity; and a gas channel in fluid communication with the nebulizer channel, wherein the gas channel is configured to direct a gas flow toward the patient cavity through the nebulizer channel, drawing a portion of the medicament from the reservoir through the nebulizer channel with the gas flow toward the patient cavity.

In certain embodiments, a ventilation mask is disclosed that comprises a mask body defining a patient cavity, the mask body comprising a patient opening in fluid communication with the patient cavity; a therapeutic gas manifold coupled to the mask body, the therapeutic gas manifold defining a therapeutic gas channel, the therapeutic gas manifold comprising a plurality of therapeutic gas ports in fluid communication with the therapeutic gas channel, wherein the plurality of therapeutic gas ports are configured to create a therapeutic gas flow within the patient cavity; and a drug delivery hub, comprising: a hub housing defining a housing volume, wherein the hub housing is disposed between the mask body and the therapeutic gas manifold; a reservoir disposed at least partially within the housing volume, wherein the reservoir comprises a reservoir volume configured to dispense a medicament; a nebulizer channel in fluid communication with the reservoir volume and configured to be in fluid communication with the patient cavity; and a gas channel in fluid communication with the nebulizer channel, wherein the gas channel is configured to direct a gas flow toward the patient cavity through the nebulizer channel, drawing a portion of the medicament from the reservoir through the nebulizer channel with the gas flow toward the patient cavity.

In certain embodiments, a method is disclosed that comprises introducing a therapeutic gas into a patient cavity of a ventilation mask via a plurality of gas ports; directing a gas flow through a nebulizer channel; drawing a portion of a medicament from a reservoir through the nebulizer channel with the gas flow, wherein the reservoir is disposed within a drug delivery hub coupled to the ventilation mask; and directing the gas flow and the portion of the medicament into the patient cavity.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 is an exploded perspective view of a ventilation mask with a drug delivery hub, in accordance with various aspects of the present disclosure.
FIG. 2 is a front elevation view of a drug delivery hub for use with the ventilation mask of FIG 1, in accordance with various aspects of the present disclosure.
FIG. 3 is a cross-sectional view of the drug delivery hub of FIG. 2, in accordance with various aspects of the present disclosure.
FIG. 4 is an exploded perspective view of a ventilation mask with a drug delivery hub, in accordance with various aspects of the present disclosure.
FIG. 5 is an exploded perspective view of a ventilation mask with a drug delivery hub, in accordance with various aspects of the present disclosure.
FIG. 6 is an exploded perspective view of a ventilation mask with a drug delivery hub, in accordance with various aspects of the present disclosure.
FIG. 7 is an exploded side elevation view of a ventilation mask with a cross-sectional view of a drug delivery hub, in accordance with various aspects of the present disclosure.
FIG. 8 is a front elevation view of a drug delivery hub for use with the ventilation mask of FIG 7, in accordance with various aspects of the present disclosure.
FIG. 9 is a cross-sectional view of the drug delivery hub of FIG. 8, in accordance with various aspects of the present disclosure.
FIG. 10 is a perspective view of a ventilation mask, in accordance with various aspects of the present disclosure.

### DETAILED DESCRIPTION

The disclosed drug delivery hub incorporates features to permit the administration of a medicament without interrupting the supplemental gas therapy or diminishing the effectiveness of the supplemental gas therapy and/or the administered drugs. The drug delivery hub can be coupled to a ventilation mask and can dispense a medicament within a flow path defined within the hub housing to generate a medicament aerosol in the immediate vicinity of the patient's airway.

The detailed description set forth below is intended as a description of various configurations of the subject technology and is not intended to represent the only configurations in which the subject technology may be practiced. The detailed description includes specific details for the purpose of providing a thorough understanding of the subject technology. However, it will be apparent to those skilled in the art that the subject technology may be practiced without these specific details. In some instances, well-known structures and components are shown in block diagram form in order to avoid obscuring the concepts of the subject technology. Like components are labeled with identical element numbers for ease of understanding. Reference numbers may have letter suffixes appended to indicate separate instances of a common element while being referred to generically by the same number without a suffix letter.

While the following description is directed to the administration of supplemental gas and/or a medicament to a patient using the disclosed drug delivery hub, it is to be understood that this description is only an example of usage and does not limit the scope of the claims. Various aspects of the disclosed drug delivery hub may be used in any application where it is desirable to administer a supplemental gas and/or a medicament.

The disclosed drug delivery hub overcomes several challenges discovered with respect to certain medication delivery devices. One challenge with certain conventional medication delivery devices, such as nebulizers, metered-dose inhalers (MDIs), and dry-powder inhalers is that such devices are primarily configured for handheld use and may obstruct or otherwise prevent the use of ventilation masks. As a result, ventilation masks are often removed during medication delivery, which may disturb the respiratory therapy and complicate caregiver workflow. In some applications, certain conventional nebulization masks can receive aerosol from a jet nebulizer via a receiving port. One challenge with certain conventional nebulization masks is that the output of the nebulizer can frequently be too locationally concentrated in a flow field that may not be appropriately directed for patient breathing (e.g. flow may be directed toward a patient's eyes), sacrificing delivery efficiency. Another challenge with certain conventional nebulization masks is that flow requirements of the nebulizer may differ from the patient's flow requirements. Further, certain conventional nebulization masks may not provide sufficient oxygen delivery during non-nebulizing periods.

Therefore, in accordance with the present disclosure, it is advantageous to provide a drug delivery hub as described herein that allows for the administration of medicament during supplemental gas (e.g. oxygen) therapy. In some embodiments, the drug delivery hub can engage with or couple with a ventilation mask, providing hands-free drug delivery. In some embodiments, the drug delivery hub can access pneumatic driving gas for nebulization though docking or sharing of gas from a ventilation mask. In some embodiments, the drug delivery hub provides distributed flow of nebulized drugs to the patient's mouth and/or nose. Further, in some embodiments, the drug delivery hub can provide simultaneous delivery of nebulized drugs and supplemental gas without the need for any additional adapters.

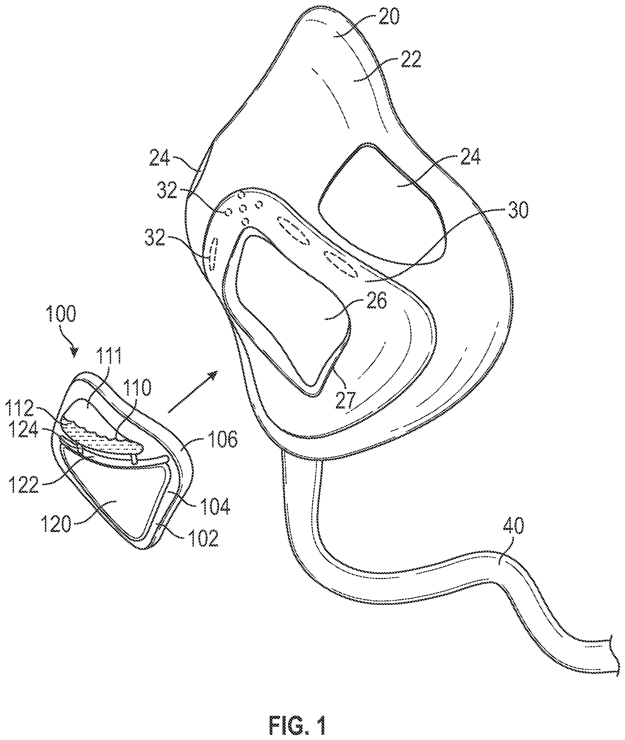

Examples of drug delivery hubs that allow for the administration of medicament during supplemental gas therapy are now described. FIG. 1 is an exploded perspective view of a ventilation mask 20 with a drug delivery hub 100, in accordance with various aspects of the present disclosure. In the depicted example, the drug delivery hub 100 can provide medicament aerosol to a patient without interrupting therapeutic gas flow from the ventilation mask 20 or otherwise removing the ventilation mask 20.

In the depicted example, the ventilation mask 20 can be utilized to administer oxygen or other supplemental gases to a patient. As illustrated, the ventilation mask 20 can be worn by the patient, defining a patient cavity over the patient's mouth and nose. The ventilation mask 20 can define an inner surface within the patient cavity and an outer surface opposite to the inner surface. The ventilation mask 20 can direct a supplemental gas, such as oxygen, via the supply line 40 to the ventilation mask 20.

In some embodiments, a gas manifold 30 can distribute the supplemental gas through the mask body 22 to the patient. In the depicted example, supplemental gases can be introduced into the patient cavity via one or more gas ports 32, formed in the gas manifold 30 disposed within the patient cavity of the mask body 22. The gas ports 32 can be disposed along the inner surface of the ventilation mask 20. In some applications, the gas ports 32 can administer high concentrations of supplemental gas to the patient cavity and ultimately to the patient.

In the depicted example, the ventilation mask 20 can have a generally open mask structure. As illustrated, the mask body 22 includes one or more vent openings 24, 26 formed therethrough. The vent openings 24, 26 can allow for access or fluid communication with the patient cavity defined by the mask body 22. In some embodiments, the mask body 22 includes three vent openings 24, 26. The upper vent openings 24 can be positioned to be adjacent to a patient's nose when the ventilation mask 20 is worn. Further, the upper vent openings 24 can be laterally spaced apart on either side of the patient's nose when the ventilation mask 20 is worn. The lower vent opening 26 can be positioned to be adjacent to a patient's mouth when the ventilation mask 20 is worn. In some embodiments, the ventilation mask 20 includes one or more of the vent openings 24, 26.

Advantageously, by utilizing one or more vent openings 24, 26, the ventilation mask 20 can allow for exhaled gases such as carbon dioxide to exit from the patient cavity of the ventilation mask, reducing the incidence of carbon dioxide rebreathing by the patient. Further, the vent openings 24, 26 can permit various tasks to be performed without removing the ventilation mask 20. Tasks can include, but are not limited to, medical procedures, eating, drinking, hygiene procedures, and/or talking. For example, the vent openings 24, 26 can allow for nasal and/or oral bronchoscopy procedures, administering medications, and/or access for drug delivery hubs as described herein. Further, the open structure of the ventilation mask 20 can increase patient comfort by accommodating various facial features and reducing patient claustrophobia.

In the depicted example, a drug delivery hub 100 can be coupled or attached to the ventilation mask 20 to deliver or administer a medicament aerosol to the patient without interrupting gas flow from the ventilation mask 20 or removing the ventilation mask 20. Medicaments can include, but are not limited to, surfactants, drugs (e.g. antibiotics, anti-viral, etc.), polymers, naturopathic remedies, and/or homeopathic remedies. The drug delivery hub 100 can be coupled or attached to the outer surface of the ventilation mask 20. Optionally, the drug delivery hub 100 can be coupled or attached to the inner surface of the ventilation mask 20. The drug delivery hub 100 can include a hub housing 102, defining a housing volume 104 therein. In some embodiments, components of the drug delivery hub 100, such as the reservoir 110, the gas channel 120, the nebulization portion 122, and the medicament channel 124 can be contained within the housing volume 104.

In some embodiments, the drug delivery hub 100 can be removably attached to the ventilation mask 20 to provide medicament aerosol when attached to the ventilation mask 20, and to allow for normal operation of the ventilation mask 20 when the drug delivery hub 100 is separated from the ventilation mask 20. In the depicted example, the drug delivery hub 100 can be attached to a vent opening 24, 26 of the ventilation mask 20. As illustrated, the drug delivery hub 100 can be coupled to the lower vent opening 26 of the ventilation mask 20. In some embodiments, the drug delivery hub 100 can have a hub housing 102 defining a continuous surface. In some applications, the hub housing 102 may at least partially obstruct the respective vent opening 24, 26 to which the drug delivery hub 100 is coupled. In some embodiments, the shape or structure of the hub housing 102 can allow for a flow to pass through the vent openings 24, 26.

Optionally, the hub perimeter 106 of the hub housing 102 can engage with the opening perimeter 27 of the vent opening 26. The hub perimeter 106 and the opening perimeter 27 can be in sealing engagement to prevent leakage of medicament aerosol and/or supplemental gas flow. In some embodiments, the hub housing 102 can deform to engage with the vent opening 26. In some embodiments, the ventilation mask 20 can deform to engage with the drug delivery hub 100. As can be appreciated, both the drug delivery hub 100 and the ventilation mask 20 can deform to allow the drug delivery hub 100 and the ventilation mask 20 to engage together and/or disengage. In some embodiments, the drug delivery hub 100 can resiliently engage or "snap in" with the ventilation mask 20.

As described herein, the drug delivery hub 100 provides nebulizing or aerosolized medicament 112 to the patient. In the depicted example, the drug delivery hub 100 receives gas flow (e.g., air, oxygen, and/or another gas or combination thereof) from a gas source. As described herein, the gas source can be an independent gas source or a gas source that is shared with the ventilation mask 20. In some embodiments, the drug delivery hub 100 can receive a gas flow from the supply line 40.

In the depicted example, gas flow can move into the gas channel 120. As can be appreciated, the gas channel 120 can be defined or formed within the housing volume 104 of the hub housing 102. In the depicted example, gas flow received by the gas channel 120 can be utilized by the drug delivery hub 100 to generate medicament 112 aerosol or nebulizer flow within the housing volume 104 to be directed to the patient cavity defined by the ventilation mask 20 or otherwise administered to the patient. As described herein, the drug delivery hub 100 can be a pneumatic aerosol generator that utilizes accelerated flow to draw medicament 112 out of a reservoir volume 111 defined by the reservoir 110 and atomizes the medicament 112 into an aerosol. Advantageously, by utilizing an aerosol generator within the housing volume 104, the medicament aerosol or nebulizer flow is generated in the immediate vicinity of the point of gas flow entry into the patient's airway. It is also contemplated that any embodiment of the present disclosure can be used with a dry powder medicament such that the accelerated gas flow causes the medicament to be pulverized and/or incorporated into the gas flow and directed toward the patient.

As can be appreciated, in some embodiments, the gas flow through the drug delivery hub 100 may be controlled to provide for increased efficiency and/or consistent droplet size production. For example, in some embodiments, the gas flow may be limited to a range between approximately 2 L/min to approximately 10 L/min to provide for increased efficiency and/or consistent droplet size production. In some embodiments, the gas flow can range between approximately 1 L/min to 20 L/min, approximately 1 L/min to 15 L/min, approximately 1 L/min to 12 L/min, approximately 1 L/min to 10 L/min, approximately 2 L/min to 20 L/min, approximately 2 L/min to 15 L/min, approximately 2 L/min to 12 L/min, approximately 2 L/min to 8 L/min, and approximately 2 L/min to 5 L/min.

In the illustrated embodiment, medicament 112 is drawn from the reservoir 110. As described herein, as gas flow moves from the gas channel 120 into the nebulization portion 122, the velocity of the gas flow within the nebulization portion 122 can increase, decreasing the fluid pressure of the gas flow within the nebulization channel (e.g., by creating a Venturi effect). As can be appreciated, the decreased fluid pressure of the gas flow within the nebulization portion 122 can draw medicament 112 into the nebulization portion 122 from the reservoir 110 via the medicament channel 124.

As illustrated, the reservoir 110 can be at least partially disposed within the housing volume 104. In some embodiments, the reservoir 110 is disposed at or near a top portion of the drug delivery hub 100. Optionally, the reservoir 110 can be disposed in any portion of the drug delivery hub 100, including the bottom portion or the lateral portions of the drug delivery hub 100.

Optionally, the reservoir 110 can be removable or removably attached to the drug delivery hub 100. The reservoir volume 111 can contain a liquid or powdered medicament 112.

After drawing the medicament 112 from the reservoir 110 into the nebulization portion 122, the force of the gas flow can accelerate and shear the medicament 112, forming droplets of medicament 112 which combine with the gas flow to form the nebulizing gas flow. As described herein, the flow rate of the gas flow can be controlled to permit the desired formation of medicament 112 droplets or aerosol appropriate for delivery into the patient's airways and/or lungs. As described here, the nebulizing gas flow can be directed into the patient cavity of the ventilation mask 20 to be inhaled by the patient's mouth and/or nose.

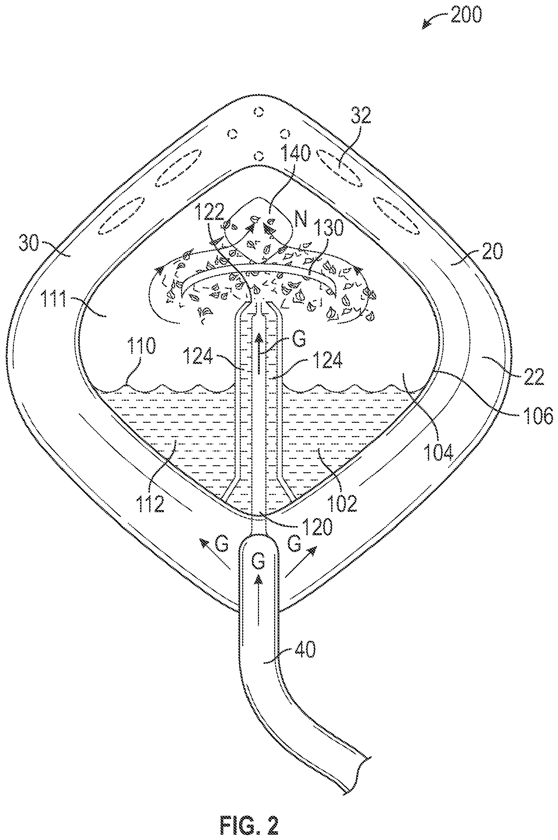

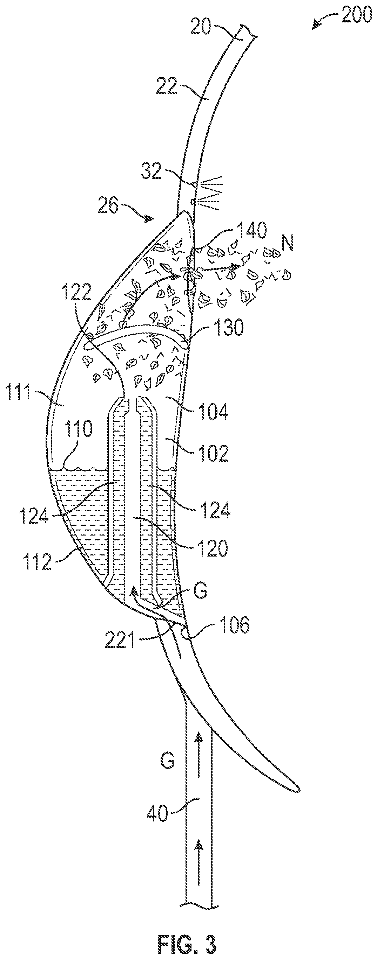

FIG. 2 is a front elevation view of a drug delivery hub 200 for use with the ventilation mask 20 of FIG 1, in accordance with various aspects of the present disclosure. FIG. 3 is a cross-sectional view of the drug delivery hub 200 of FIG. 2, in accordance with various aspects of the present disclosure. The drug delivery hub 200 can include features that are similar to drug delivery hub 100 and can operate in a similar manner. Unless noted, similar features of drug delivery hub 200 can be referred to with the same reference numerals as discussed with reference to drug delivery hub 100. With reference to FIGS. 2 and 3, the drug delivery hub 200 facilitates the nebulization of medicament 112 by drawing the medicament from a reservoir 110 toward a nebulization portion 122 through capillary action.

In the depicted example, medicament 112 is stored in a reservoir 110 defined within the housing volume 104. As illustrated, the reservoir 110 can utilize the housing volume 104 as the reservoir volume 111, storing medicament 112 in a lower portion of the housing volume 104. In some embodiments, the reservoir volume 111 is pre-filled with medicament 112. Optionally, medicament 112 can be introduced or metered into the reservoir 110 through a medicament delivery line. In some embodiments, the reservoir 110 can include an access port to allow manual administration of medicament 112 into the reservoir 110 to fill and/or refill with reservoir 110. Further, the access port can allow for the introduction of an ampule or a cartridge containing a medicament. In some embodiments, the reservoir 110 can be disposed at least partially or completely within the housing volume 104.

In the depicted example, gas flow G is introduced into the drug delivery hub 200 from the gas supply line 40. As illustrated, the gas flow G can be introduced into the drug delivery hub 200 and/or the gas channel 120 at a bottom portion of the drug delivery hub 200. In some embodiments, the gas supply line 40 is attached to the drug delivery hub 200 by an access port 221. The access port 221 can include a self-sealing membrane and/or a luer connection to seal the access port 221 when the drug delivery hub 200 is not connected to the gas supply line 40. During operation, the self-sealing membrane or luer connection can allow fluid communication between the gas supply line 40 and the gas channel 120. Optionally, the gas flow G from the gas supply line 40 can be shared between the drug delivery hub 200 and the gas manifold 30.

In the depicted example, the gas flow G can be directed upward through the gas channel 120 toward an upper portion of the drug delivery hub 200. In some embodiments, the gas channel 120 is centrally located within the housing volume 104. During operation, the drug delivery hub 200 directs gas flow G from the gas channel 120 toward the nebulizing portion 122. As illustrated, the nebulizing portion 122 of the gas channel 120 can have a constricting geometry or otherwise reduced diameter relative to the gas channel 120 to increase the velocity of the gas flow G. As can be appreciated, as the velocity of the gas flow G is increased within the nebulizing portion 122, the fluid pressure of the gas flow G within the nebulizing portion 122 is reduced (e.g., a Venturi effect). As can be appreciated, the decreased fluid pressure of the gas flow within the nebulization portion 122 can draw medicament 112 from the medicament channel 124 into the nebulizing portion 122.

In some embodiments, the medicament 112 can be drawn upward from the reservoir 110 toward the nebulizing portion 122 through a medicament channel 124. The medicament channel 124 can utilize capillary action to draw the medicament 112 upward. Optionally, the medicament channel 124 can have a generally annular shape formed around the gas channel 120. In some embodiments, the medicament channel 124 can extend into the lower or base portion of the reservoir 110, allowing the medicament channel 124 to draw medicament 112 from the reservoir 110 as the medicament 112 is consumed or nebulized.

As the medicament 112 is drawn into the nebulizing portion 122 by the gas flow G, the force of the high-speed gas flow G can accelerate and shear the medicament 112, forming droplets of medicament 112 which combine with the gas flow G to form the nebulizing gas flow N. As described herein, the flow rate of the gas flow G can be controlled to permit the desired formation of medicament 112 droplets or aerosol appropriate for delivery into the patient's airways and/or lungs.

In some embodiments of the present disclosure, the flow rate of the gas flow G can be affected by the cross-sectional width of the gas channel 120. In some embodiments, the gas channel 120 can include a constriction or narrower portion relative to the gas supply line 40. In some embodiments, a removable constriction can be positioned in the gas channel 120 to achieve a specific gas flow G rate. The removable constriction (not shown) can be a baffle, an orifice or an o-ring positioned in the gas channel 120 at the access port 221.

In the depicted example, the drug delivery hub 200 includes a baffle 130 to promote disintegration of the liquid medicament 112 within the nebulizing gas flow N. As illustrated, the baffle 130 can be disposed within the housing volume 104 such that nebulizing gas flow N impinges upon the baffle 130. The baffle 130 can be positioned between the nebulizing portion 122 and the flow outlet 140, preventing direct flow of nebulizing gas flow N into the flow outlet 140. Further, the baffle 130 can allow larger droplets to be captured and redirected toward the reservoir 110.

In some embodiments, at least one baffle 130 can be disposed adjacent to the exit of the nebulizing portion 122. Optionally, the baffle 130 can be spaced apart from the exit of the nebulizing portion 122. In some embodiments, the baffle 130 can generally have a shield or umbrella shape. As can be appreciated, the baffle 130 can be formed as any suitable shape. As can be appreciated, the positioning and the geometry of the baffle 130 can be adjusted relative to the nebulizing portion 122 to promote droplet formation and additional pulverization within the nebulizing gas flow N.

During operation, the nebulizing gas flow N can flow into the patient cavity defined by the ventilation mask 20 through the flow outlet 140. As can be appreciated, the nebulizing gas flow N can mix with the supplemental gas flow provided by the gas ports 32 to be inhaled by the patient.

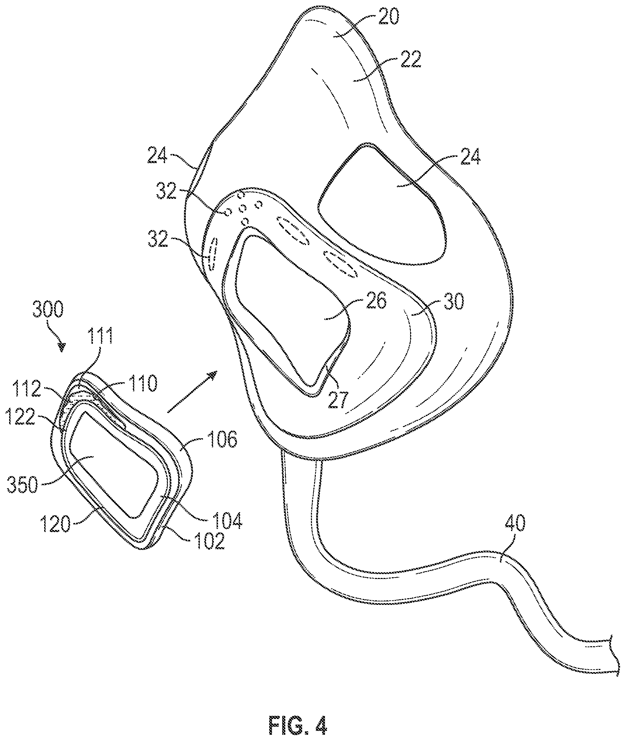

FIG. 4 is an exploded perspective view of a ventilation mask 20 with a drug delivery hub 300, in accordance with various aspects of the present disclosure. The drug delivery hub 400 can include features that are similar to drug delivery hub 100 and can operate in a similar manner. Unless noted, similar features of drug delivery hub 400 can be referred to with the same reference numerals as discussed with reference to drug delivery hub 100. In the depicted example, the drug delivery hub 300 can have an opening 350 to allow the ventilation mask 20 to permit access into the patient cavity through the lower vent opening 26. Advantageously, the opening 350 can allow the patient to continue to speak, drink, or perform other activities during nebulization procedures.

As illustrated, the drug delivery hub 300 can have a generally ring-like structure forming the opening 350 therethrough. The opening 350 can be defined by an inner surface of the drug delivery hub 300. The drug delivery hub 300 can have a continuous perimeter defining the opening 350 therein, or can have a semi-continuous perimeter, such as a U-shape, defining the opening 350 therein. In the depicted example, the components of the drug delivery hub 300 are disposed around the perimeter of the opening 350, permitting the formation of the opening 350 and the nebulizing functionality described herein. In some embodiments, the gas channel 120 can be formed generally around the opening 350. Optionally, the reservoir 110 can be disposed around the opening 350 or away from the opening 350.

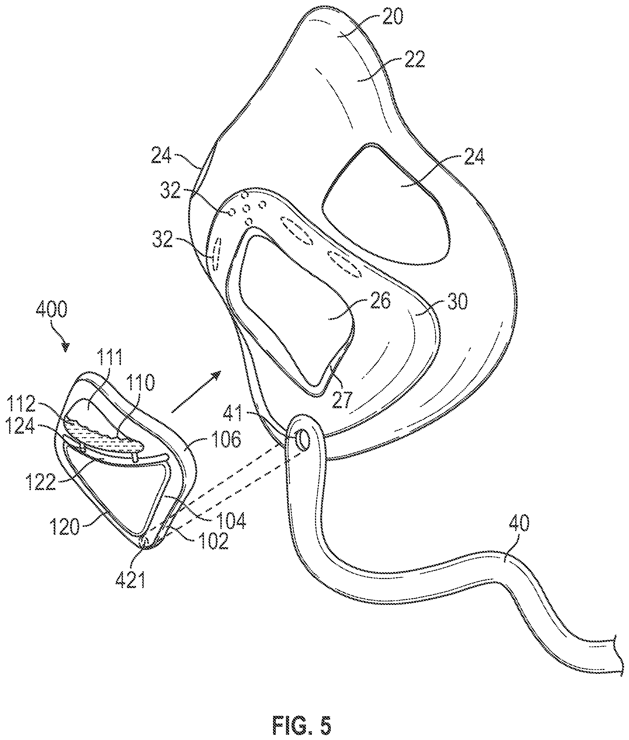

FIG. 5 is an exploded perspective view of a ventilation mask 20 with a drug delivery hub 400, in accordance with various aspects of the present disclosure. The drug delivery hub 400 can include features that are similar to drug delivery hub 100 and can operate in a similar manner. Unless noted, similar features of drug delivery hub 400 can be referred to with the same reference numerals as discussed with reference to drug delivery hub 100. In the depicted example, the gas channel 120 of the drug delivery hub 400 receives gas flow from the supply line 40. As illustrated, the drug delivery hub 400 can include a port 421 to receive gas flow from a port 41 formed in the supply line 40. As can be appreciated, the port 421 can be aligned to engage with the port 41 when the drug delivery hub 400 is engaged or otherwise coupled with the ventilation mask 20. Optionally, the port 421 can be formed as a part of the hub housing 102.

In some embodiments, gas within the supply line 40 can be shared between the drug delivery hub 400 and the ventilation mask 20. As can be appreciated, a portion of the gas flow can exit the supply line 40 at the port 41 and another portion of gas flow can continue into the ventilation mask 20. In some embodiments, the port 41 and/or the port 421 can include a self-sealing membrane or a luer connection to allow for the port 41 and/or the port 421 to seal when not in use.

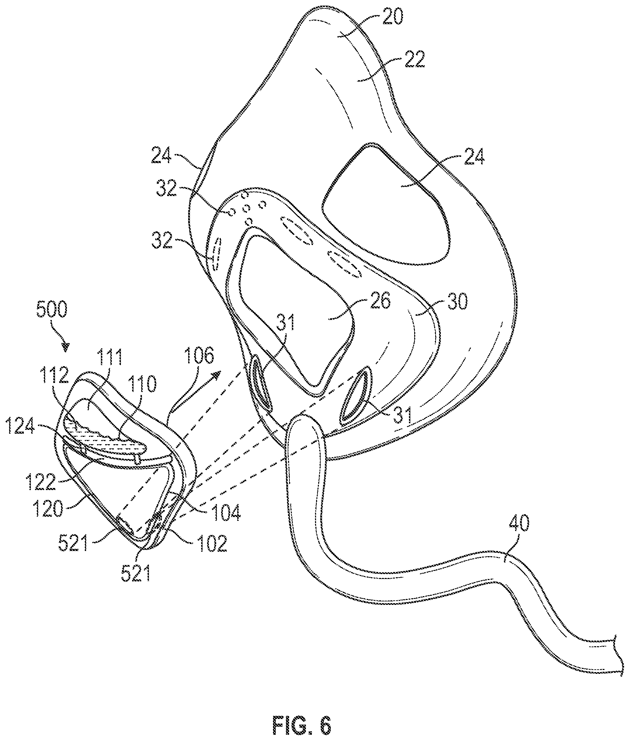

FIG. 6 is an exploded perspective view of a ventilation mask 20 with a drug delivery hub 500, in accordance with various aspects of the present disclosure. In the depicted example, the gas channel 120 of the drug delivery hub 500 receives gas flow from the ports 31 formed on the ventilation mask 20. The drug delivery hub 500 can include features that are similar to drug delivery hub 100 and can operate in a similar manner. Unless noted, similar features of drug delivery hub 500 can be referred to with the same reference numerals as discussed with reference to drug delivery hub 100. As illustrated, the drug delivery hub 500 can include ports 521 to receive gas flow from the ventilation mask 20. As can be appreciated, the ports 521 can be aligned to engage with the ports 31 when the drug delivery hub 500 is engaged or otherwise coupled with the ventilation mask 20. Optionally, the ports 521 can be formed as a part of the hub housing 102.

In some embodiments, gas from the ventilation mask 20 and/or the gas manifold 30 can be shared with the drug delivery hub 500. As can be appreciated, gas can flow into the ventilation mask 20 from the supply line 40 to deliver supplemental gas flow to the patient via the gas ports 32 and deliver gas flow to the drug delivery hub 500 via the ports 31. In some embodiments, the ports 31 and/or the ports 521 can include a self-sealing membrane or a luer connection to allow for the port 31 and/or the port 521 to seal when not in use.

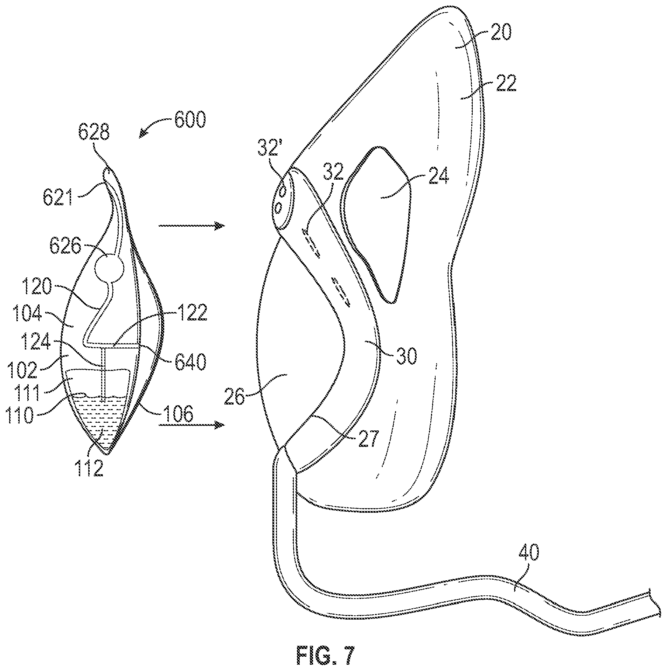

FIG. 7 is an exploded side elevation view of a ventilation mask 20 with a cross-sectional view of a drug delivery hub 600, in accordance with various aspects of the present disclosure. The drug delivery hub 600 can include features that are similar to drug delivery hub 100 and can operate in a similar manner. Unless noted, similar features of drug delivery hub 600 can be referred to with the same reference numerals as discussed with reference to drug delivery hub 100. In the depicted example, the drug delivery hub 600 can receive gas flow for nebulization from the gas ports 32' of the ventilation mask 20 that are typically used for delivering supplemental gas flow to the patient. As illustrated, when the drug delivery hub 600 is coupled to the ventilation mask 20, the drug delivery hub 600 is shaped or otherwise configured to allow the gas channel 120 to be in fluid communication with one or more of the gas ports 32' of the ventilation mask 20. Optionally, the gas channel 120 can include a flow feature 626 to adjust the flow within the gas channel 120. As illustrated, nebulizing flow can enter the patient cavity via the outlet 640.

In some embodiments, the reservoir 110 can be disposed toward a lower portion of the hub housing 102. In some embodiments, the reservoir 110 can be disposed below the gas channel 120, the medicament channel 124, and the nebulization portion 122. Advantageously, by locating the reservoir 110 below other components of the drug delivery hub 600, the hub housing 102 may be configured to be more compact.

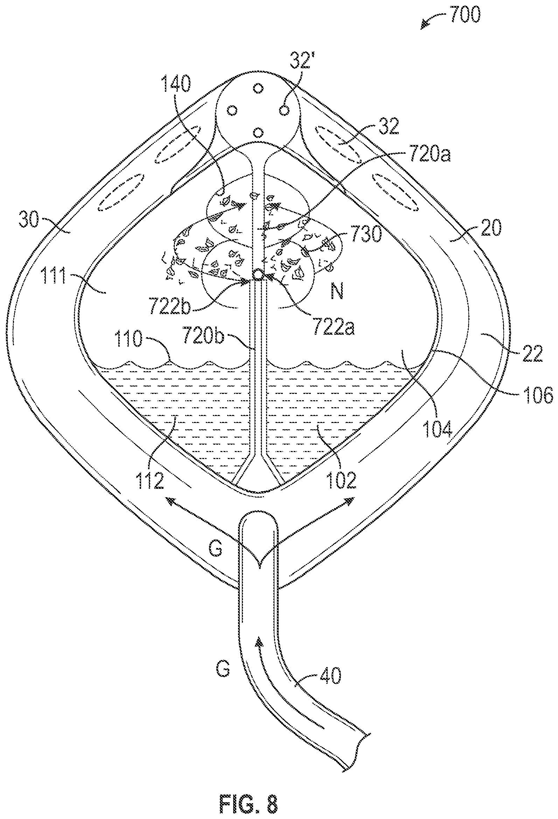

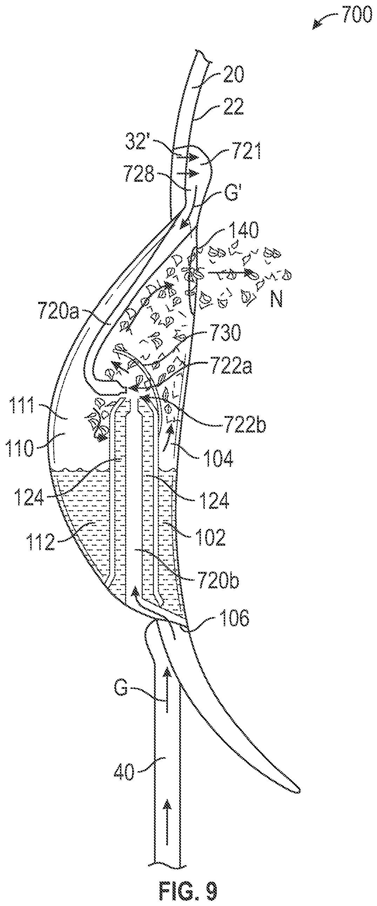

FIG. 8 is a front elevation view of a drug delivery hub 700 for use with the ventilation mask 20 of FIG. 7, in accordance with various aspects of the present disclosure. FIG. 9 is a cross-sectional view of the drug delivery hub 700 of FIG. 8, in accordance with various aspects of the present disclosure. The drug delivery hub 700 can include features that are similar to drug delivery hub 600 and can operate in a similar manner. Unless noted, similar features of drug delivery hub 700 can be referred to with the same reference numerals as discussed with reference to drug delivery hub 600. With reference to FIGS. 8 and 9, the drug delivery hub 700 facilitates nebulization by drawing gas flow from gas ports 32' of the ventilation mask 20. Similar to drug delivery hub 200, the medicament 112 can be stored in a reservoir 110 defined within the housing volume 104.

In the depicted example, gas flow G' is introduced into the drug delivery hub 700 from the gas ports 32' of the ventilation mask 20. In the depicted example, the access port extension 728 of the drug delivery hub 700 extends into an inner portion of the ventilation mask 20 to align or otherwise engage the access ports 721 of the access port extension 728 with one or more of the gas ports 32' of the ventilation mask 20. Optionally, the access port extension 728 can seal against an inner surface of the ventilation mask 20 adjacent to the gas ports 32'. The access ports 721 can include a self-sealing membrane. Advantageously, the access ports 721 can capture flow from a portion of the gas ports 32', allowing for flow to be split between the drug delivery hub 700 and gas flow into the patient cavity.

In the depicted example, the gas flow G' can be directed downward through a gas channel 720a toward a lower portion of the drug delivery hub 700. During operation, the gas channel 720a can direct gas flow G' toward the nebulizing portion 722a. As illustrated, the nebulizing portion 722a of the gas channel 720a can have a constricting geometry or otherwise reduced diameter relative to the gas channel 720a to increase the velocity of the gas flow G'. As can be appreciated, as the velocity of the gas flow G' is increased within the nebulizing portion 722a, the fluid pressure of the gas flow G' within the nebulizing portion 722a is reduced (e.g., a Venturi effect). As can be appreciated, the decreased fluid pressure of the gas flow within the nebulization portion 722a can draw medicament 112 from the medicament channel 124 toward the nebulizing portion 722a. In some embodiments, the gas channel 720a can be directed toward the patient or away from the patient.

Additionally or alternatively, the gas flow G can also be introduced into the drug delivery hub 700 by a gas channel 720b at a bottom portion of the drug delivery hub 700. In some embodiments, the gas supply line 40 is attached to the drug delivery hub 700 by an access port. In some embodiments, the gas flow G from the gas supply line 40 can be shared between the drug delivery hub 700 and the gas manifold 30. It should be understood that the drug delivery hub of the present disclosure can be configured with either a gas channel 720a to direct a gas G' toward a nebulizing portion 722a, or a gas channel 720b to direct a gas G toward a nebulizing portion 722b. It should also be understood that the drug delivery hub of the present disclosure can be configured with a gas channel 720a and a gas channel 720b to direct a gas to a nebulizing portions 722a, 722b.

In the depicted example, the gas flow G can be directed upward through the gas channel 720b toward an upper portion of the drug delivery hub 700. During operation, the drug delivery hub 700 directs gas flow G from the gas channel 720b toward the nebulizing portion 722b. As illustrated, the nebulizing portion 722b of the gas channel 720b can have a constricting geometry or otherwise reduced diameter relative to the gas channel 720b to increase the velocity of the gas flow G. As can be appreciated, as the velocity of the gas flow G is increased within the nebulizing portion 722b, the fluid pressure of the gas flow G within the nebulizing portion 722b is reduced (e.g., a Venturi effect). In addition to the gas flow G from the gas channel 720a the decreased fluid pressure of the gas flow within the nebulization portion 722b can draw medicament 112 from the medicament channel 124 into the nebulizing portion 722b.

In some embodiments, the medicament 112 can be drawn upward from the reservoir 110 toward the nebulizing portion 722a, 722b through a medicament channel 124. The medicament channel 124 can utilize capillary action to draw the medicament 112 upward. Optionally, the medicament channel 124 can have a generally annular shape formed around the gas channel 720b. In some embodiments, the medicament channel 124 can extend into the lower or base portion of the reservoir 110, allowing the medicament channel 124 to draw medicament 112 from the reservoir 110 as the medicament 112 is consumed or nebulized.

As the medicament 112 is drawn toward the nebulizing portion 722a, 722b by the gas flow G, the force of the high-speed gas flow G can accelerate and shear the medicament 112, forming droplets of medicament 112 that combine with the gas flow G to form the nebulizing gas flow N. As described herein, the flow rate of the gas flow G can be controlled to permit the desired formation of medicament 112 droplets or aerosol appropriate for delivery into the patient's airways and/or lungs.

In the depicted example, the drug delivery hub 700 includes a baffle 730 to promote atomization of the liquid medicament 112, or pulverization of a dry medicament, within the nebulizing gas flow N. As illustrated, the baffle 730 can be disposed within the housing volume 104 such that nebulizing gas flow N impinges upon the baffle 730. The baffle 730 can be positioned between the nebulizing portion 722a, 722b and the flow outlet 140, preventing direct flow of nebulizing gas flow N into the flow outlet 140. Further, the baffle 730 can allow larger droplets to be captured and redirected toward the reservoir 110. In some embodiments, at least one baffle 730 can be disposed adjacent to the exit of the nebulizing portion 722a, 722b. Optionally, the baffle 730 can be spaced apart from the exit of the nebulizing portion 722a, 722b. In some embodiments, the baffle 730 can generally have a shield or umbrella shape. As can be appreciated, the baffle 730 can be formed as any suitable shape. As can be appreciated, the positioning and the geometry of the baffle 730 can be adjusted relative to the nebulizing portion 722a, 722b to promote droplet formation and additional pulverization within the nebulizing gas flow N. Optionally, the walls of the hub housing 102 can be shaped to function as a baffle.

During operation, the nebulizing gas flow N can flow into the patient cavity defined by the ventilation mask 20 through the flow outlet 140. As can be appreciated, the nebulizing gas flow N can mix with the supplemental gas flow provided by the gas ports 32 to be inhaled by the patient.

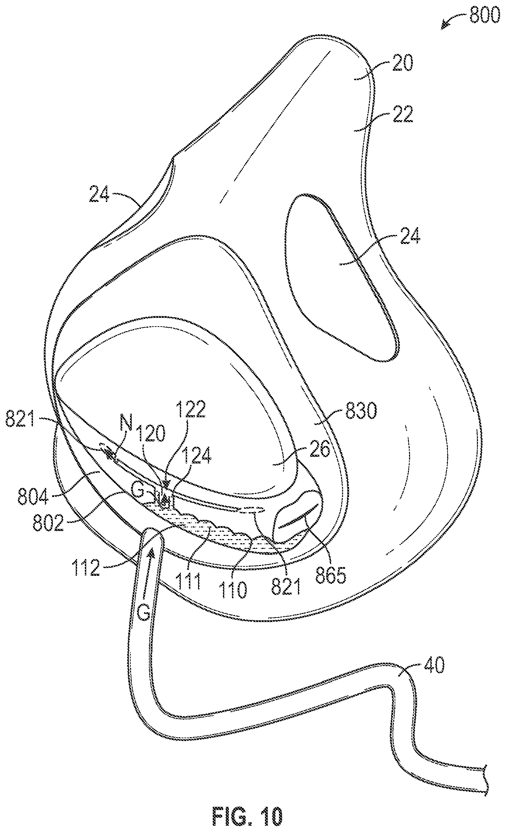

FIG. 10 is a perspective view of a ventilation mask 800, in accordance with various aspects of the present disclosure. The ventilation mask 800 can include features that are similar to drug delivery hub 100 and can operate in a similar manner. Unless noted, similar features of ventilation mask 800 can be referred to with the same reference numerals as discussed with reference to drug delivery hub 100. In the depicted example, the ventilation mask 800 includes an integrated nebulizer to provide supplemental gas flow and/or nebulizing gas flow as required.

In the depicted example, medicament 112 is stored in a reservoir 110 defined within the nebulizer manifold 830. As illustrated, the reservoir 110 can utilize the nebulizer manifold 830 as the reservoir volume 111, storing medicament 112 in a lower portion of the nebulizer manifold 830. In some embodiments, the reservoir volume 111 is pre-filled with medicament 112. In some embodiments, the nebulizer manifold 830 can include a fill port 865 to allow manual administration of medicament 112 into the reservoir 110 to fill and/or refill reservoir 110. Advantageously, the ventilation mask 800 can function as a ventilation mask when the reservoir 110 is empty and provide nebulization functionality when the reservoir 810 is filled with medicament 112.

In the depicted example, gas flow G is introduced into the ventilation mask 800 from the gas supply line 40. As illustrated, the gas flow G can be introduced into the ventilation mask 800 to delivery gas flow G for nebulization and for supplemental gas therapy. Gas flow G can be shared between nebulization and supplemental gas therapy. In some embodiments, the gas flow G, or a portion thereof, is directed into the gas channel 120 and then toward the patient via access ports 821.

During operation, the ventilation mask 800 directs gas flow G through a portion of the gas channel 120 having a constricting geometry or otherwise reduced diameter relative to another portion of the gas channel to increase the velocity of the gas flow G. As can be appreciated, as the velocity of the gas flow G is increased, the fluid pressure of the gas flow G is reduced (e.g., a Venturi effect). The decreased fluid pressure of the gas flow within the gas channel can draw medicament 112 toward the gas channel 120.

In some embodiments, the medicament 112 can be drawn, by gas flow G through the gas channel 120, from the reservoir 110 through a medicament channel 124, similar to the gas channel 720b and the medicament channel 124 described with reference to Figure 9. In some embodiments, the medicament channel 124 can extend into the lower or base portion of the reservoir 110, allowing the medicament channel 124 to draw medicament 112 from the reservoir 110 as the medicament 112 is consumed or nebulized.

As the medicament 112 is drawn into the nebulizing portion 122 by the gas flow G, the force of the high-speed gas flow G can accelerate and shear the medicament 112, forming droplets of medicament 112 which combine with the gas flow G to form the nebulizing gas flow.

During operation, the nebulizing gas flow N can move into the patient cavity defined by the ventilation mask 800 through the access ports 821. As can be appreciated, the nebulizing gas flow N can mix with the supplemental gas flow provided by the gas ports to be inhaled by the patient.

### Further Considerations

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

In one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

## Claims

1. A drug delivery hub (100, 200, 300, 400, 500, 600, 700, 800), comprising:
a hub housing (102) defining a housing volume (104), wherein the hub housing is configured to directly couple with a mask (20) defining a patient cavity;
a reservoir (110) disposed at least partially within the housing volume, wherein the reservoir comprises a reservoir volume (111) configured to dispense a medicament (112);
a nebulizer channel (124) in fluid communication with the reservoir volume and configured to be in fluid communication with the patient cavity when the hub is coupled with the mask; and
a gas channel (120, 720a, 720b) in fluid communication with the nebulizer channel, wherein the gas channel is configured to receive a gas flow (G) from a port (31, 32, 32', 41) on the mask and direct the gas flow toward the patient cavity through the nebulizer channel, drawing a portion of the medicament from the reservoir through the nebulizer channel with the gas flow toward the patient cavity.

2. The drug delivery hub of Claim 1, wherein the gas channel comprises an access port (221, 721, 821) to receive the gas flow (G).

3. The drug delivery hub of Claim 2, wherein the access port (221, 721, 821) is configured to engage with a self-sealing membrane or luer connection.

4. The drug delivery hub of Claim 1, wherein the hub housing (102) comprises an inner surface that defines a passage (140, 640) through the hub, such that, when the hub is coupled with the mask, the passage extends through the hub to the patient cavity.

5. The drug delivery hub of Claim 1, wherein, when the hub is coupled with a mask, the hub is configured to direct the gas through the hub and back to the mask.

6. The drug delivery hub of Claim 1, wherein the hub is configured to be removably coupled to a mask.

7. A ventilation mask (20), comprising:
a mask body (22) defining a patient cavity, the mask body comprising a patient opening in fluid communication with the patient cavity; and
the drug delivery hub (100, 200, 300, 400, 500, 600, 700, 800) of Claim 1.

8. The ventilation mask of Claim 7, wherein the drug delivery hub can be removably coupled to the mask body such that the ventilation mask can provide a gas to the patient cavity when the drug delivery hub is coupled to the mask body and when the drug delivery hub is uncoupled from the mask body.

9. The ventilation mask of Claim 7, further comprising:
at least one vent opening (24, 26) formed through the mask body (22), the at least one vent opening in fluid communication with the patient cavity, wherein the at least one vent opening is disposed generally opposite to the patient opening, and the hub housing (102) is coupled to the at least one vent opening.

10. The ventilation mask of Claim 9, wherein the hub housing (102) comprises a continuous surface and at least partially obstructs the at least one vent opening (24, 26).

11. The ventilation mask of Claim 7, further comprising:
a therapeutic gas manifold (30) coupled to the mask body (22), the therapeutic gas manifold defining a therapeutic gas channel, the therapeutic gas manifold comprising a plurality of therapeutic gas ports (31, 32, 32') in fluid communication with the therapeutic gas channel, wherein the plurality of therapeutic gas ports are configured to create a therapeutic gas flow within the patient cavity.

12. The ventilation mask of Claim 11, wherein, when the hub is coupled with the mask, a portion of the hub obstructs at least a portion of the plurality of therapeutic gas ports (31, 32, 32') to scavenge a gas exiting therefrom and direct the scavenged gas into the gas channel of the hub.

13. A method comprising:
introducing a therapeutic gas into a patient cavity of a ventilation mask (20) via a plurality of gas ports (31, 32, 32');
directing a gas flow (G) from a port (31, 32, 32', 41) on the mask through a nebulizer channel (124) of a drug delivery hub (100, 200, 300, 400, 500, 600, 700, 800) coupled with the ventilation mask;
drawing a portion of a medicament (112) from a reservoir (110) through the nebulizer channel (124) with the gas flow, wherein the reservoir is disposed within the drug delivery hub; and
directing the gas flow and the portion of the medicament into the patient cavity of the ventilation mask.

14. The method of Claim 13, further comprising attaching the drug delivery hub (100, 200, 300, 400, 500, 600, 700, 800) to the ventilation mask (20).

15. The method of Claim 14, wherein attaching the drug delivery hub (100, 200, 300, 400, 500, 600, 700, 800) comprises attaching the drug delivery hub to a vent opening (24, 26) of the ventilation mask.

## Patentansprüche

1. Medikamentenverabreichungsaufsatz (100, 200, 300, 400, 500, 600, 700, 800), aufweisend:
ein Aufsatzgehäuse (102), das ein Gehäusevolumen (104) definiert, wobei das Aufsatzgehäuse dafür ausgelegt ist, direkt mit einer Maske (20) verbunden zu werden, die eine Patientenhöhlung definiert;
ein Reservoir (110), das zumindest teilweise innerhalb des Gehäusevolumens angeordnet ist, wobei das Reservoir ein Reservoirvolumen (111) aufweist, das dafür ausgelegt ist, ein Medikament (112) abzugeben;
einen Vernebelungskanal (124), der mit dem Reservoirvolumen in Fluidverbindung steht und dafür ausgelegt ist, mit der Patientenhöhlung in Fluidverbindung zu stehen, wenn der Aufsatz mit der Maske verbunden ist; und
einen Gaskanal (120, 720a, 720b), der mit dem Vernebelungskanal in Fluidverbindung steht, wobei der Gaskanal dafür ausgelegt ist, einen Gasstrom (G) aus einer Öffnung (31, 32, 32', 41) an der Maske aufzunehmen und den Gasstrom durch den Vernebelungskanal zur Patientenhöhlung zu leiten, wobei ein Teil des Medikaments aus dem Reservoir durch den Vernebelungskanal mit dem Gasstrom zur Patientenhöhlung gesaugt wird.

2. Medikamentenverabreichungsaufsatz nach Anspruch 1, wobei der Gaskanal eine Zugangsöffnung (221, 721, 821) zur Aufnahme des Gasstroms (G) aufweist.

3. Medikamentenverabreichungsaufsatz nach Anspruch 2, wobei die Zugangsöffnung (221, 721, 821) dafür ausgelegt ist, mit einer selbstabdichtenden Membran oder einem Luer-Anschluss in Eingriff zu sein.

4. Medikamentenverabreichungsaufsatz nach Anspruch 1, wobei das Aufsatzgehäuse (102) eine Innenfläche aufweist, die einen Durchgang (140, 640) durch den Aufsatz definiert, so dass, wenn der Aufsatz mit der Maske verbunden ist, der Durchgang sich durch den Aufsatz zur Patientenhöhlung erstreckt.

5. Medikamentenverabreichungsaufsatz nach Anspruch 1, wobei, wenn der Aufsatz mit einer Maske verbunden ist, der Aufsatz dafür ausgelegt ist, das Gas durch den Aufsatz und zurück zur Maske zu leiten.

6. Medikamentenverabreichungsaufsatz nach Anspruch 1, wobei der Aufsatz dafür ausgelegt ist, abnehmbar mit einer Maske verbunden zu werden.

7. Beatmungsmaske (20), aufweisend:
einen Maskenkorpus (22), der eine Patientenhöhlung definiert, wobei der Maskenkorpus eine Patientenöffnung aufweist, die mit der Patientenhöhlung in Fluidverbindung steht; und
den Medikamentenverabreichungsaufsatz (100, 200, 300, 400, 500, 600, 700, 800) nach Anspruch 1.

8. Beatmungsmaske nach Anspruch 7, wobei der Medikamentenverabreichungsaufsatz abnehmbar mit dem Maskenkorpus verbunden werden kann, derart, dass die Beatmungsmaske der Patientenhöhlung ein Gas bereitstellen kann, wenn der Medikamentenverabreichungsaufsatz mit dem Maskenkorpus verbunden ist und der Medikamentenverabreichungsaufsatz vom Maskenkorpus abgekoppelt ist.

9. Beatmungsmaske nach Anspruch 7, darüber hinaus aufweisend:
mindestens eine Entlüftungsöffnung (24, 26), die durch den Maskenkorpus (22) hindurch ausgebildet ist, wobei die mindestens eine Entlüftungsöffnung mit der Patientenhöhlung in Fluidverbindung steht, wobei die mindestens eine Entlüftungsöffnung allgemein gegenüberliegend zur Patientenöffnung angeordnet ist und das Aufsatzgehäuse (102) mit der mindestens einen Entlüftungsöffnung verbunden ist.

10. Beatmungsmaske nach Anspruch 9, wobei das Aufsatzgehäuse (102) eine durchgehende Oberfläche aufweist und die mindestens eine Entlüftungsöffnung (24, 26) zumindest teilweise blockiert.

11. Beatmungsmaske nach Anspruch 7, darüber hinaus aufweisend:
einen Therapiegasverteiler (30), der mit dem Maskenkorpus (22) verbunden ist, wobei der Therapiegasverteiler einen Therapiegaskanal definiert, wobei der Therapiegasverteiler mehrere Therapiegasöffnungen (31, 32, 32') aufweist, die mit dem Therapiegaskanal in Fluidverbindung stehen, wobei die mehreren Therapiegasöffnungen dafür ausgelegt sind, einen Therapiegasstrom innerhalb der Patientenhöhlung zu erzeugen.

12. Beatmungsmaske nach Anspruch 11, wobei, wenn der Aufsatz mit der Maske verbunden ist, ein Teil des Aufsatzes zumindest einen Teil der mehreren Therapiegasöffnungen (31, 32, 32') blockiert, um ein aus diesen austretendes Gas zu reinigen und das gereinigte Gas in den Gaskanal des Aufsatzes zu leiten.

13. Verfahren, umfassend:
Einleiten eines Therapiegases in eine Patientenhöhlung einer Beatmungsmaske (20) über mehrere Gasöffnungen (31, 32, 32');
Leiten eines Gasstroms (G) von einer Öffnung (31, 32, 32', 41) an der Maske durch einen Vernebelungskanal (124) eines Medikamentenverabreichungsaufsatzes (100, 200, 300, 400, 500, 600, 700, 800), der mit der Beatmungsmaske verbunden ist;
Saugen eines Teils eines Medikaments (112) von einem Reservoir (110) durch den Vernebelungskanal (124) mit dem Gasstrom, wobei das Reservoir innerhalb des Medikamentenverabreichungsaufsatzes angeordnet ist; und
Leiten des Gasstroms und des Teils des Medikaments in die Patientenhöhlung der Beatmungsmaske.

14. Verfahren nach Anspruch 13, darüber hinaus umfassend, den Medikamentenverabreichungsaufsatz (100, 200, 300, 400, 500, 600, 700, 800, 900) an der Beatmungsmaske (20) anzubringen.

15. Verfahren nach Anspruch 14, wobei das Anbringen des Medikamentenverabreichungsaufsatzes (100, 200, 300, 400, 500, 600, 700, 800) umfasst, den Medikamentenverabreichungsaufsatz an einer Entlüftungsöffnung (24, 26) der Beatmungsmaske anzubringen.

## Revendications

1. Embout de distribution de médicament (100, 200, 300, 400, 500, 600, 700, 800), comprenant :
un boîtier d'embout (102) définissant un volume de boîtier (104), sachant que le boîtier d'embout est configuré pour se coupler directement avec un masque (20) définissant une cavité de patient ;
un réservoir (110) disposé au moins en partie à l'intérieur du volume de boîtier, sachant que le réservoir comprend un volume de réservoir (111) configuré pour administrer un médicament (112) ;
un canal de nébuliseur (124) en communication fluidique avec le volume de réservoir et configuré pour être en communication fluidique avec la cavité de patient lorsque l'embout est couplé avec le masque ; et
un canal de gaz (120, 720a, 720b) en communication fluidique avec le canal de nébuliseur, sachant que le canal de gaz est configuré pour recevoir un flux de gaz (G) depuis un orifice (31, 32, 32', 41) sur le masque et diriger le flux de gaz vers la cavité de patient à travers le canal de nébuliseur, soutirant une partie du médicament depuis le réservoir à travers le canal de nébuliseur avec le flux de gaz vers la cavité de patient.

2. L'embout de distribution de médicament de la revendication 1, sachant que le canal de gaz comprend un orifice d'accès (221, 721, 821) pour recevoir le flux de gaz (G).

3. L'embout de distribution de médicament de la revendication 2, sachant que l'orifice d'accès (221, 721, 821) est configuré pour se mettre en prise avec une membrane auto-étanchéifiante ou un raccord Luer.

4. L'embout de distribution de médicament de la revendication 1, sachant que le boîtier d'embout (102) comprend une surface intérieure qui définit un passage (140, 640) à travers l'embout, de telle sorte que, lorsque l'embout est couplé avec le masque, le passage s'étende à travers l'embout vers la cavité de patient.

5. L'embout de distribution de médicament de la revendication 1, sachant que, lorsque l'embout est couplé avec un masque, l'embout est configuré pour diriger le gaz à travers l'embout et en retour vers le masque.

6. L'embout de distribution de médicament de la revendication 1, sachant que l'embout est configuré pour être couplé de manière amovible à un masque.

7. Masque de ventilation (20), comprenant :
un corps de masque (22) définissant une cavité de patient, le corps de masque comprenant une ouverture de patient en communication fluidique avec la cavité de patient ; et
l'embout de distribution de médicament (100, 200, 300, 400, 500, 600, 700, 800) de la revendication 1.

8. Le masque de ventilation de la revendication 7, sachant que l'embout de distribution de médicament peut être couplé de manière amovible au corps de masque de telle sorte que le masque de ventilation puisse fournir un gaz à la cavité de patient lorsque l'embout de distribution de médicament est couplé au corps de masque et lorsque l'embout de distribution de médicament est découplé du corps de masque.

9. Le masque de ventilation de la revendication 7, comprenant en outre :
au moins une ouverture d'évent (24, 26) formée à travers le corps de masque (22), l'au moins une ouverture d'évent étant en communication fluidique avec la cavité de patient, sachant que l'au moins une ouverture d'évent est disposée de manière sensiblement opposée à l'ouverture de patient, et le boîtier d'embout (102) est couplé à l'au moins une ouverture d'évent.

10. Le masque de ventilation de la revendication 9, sachant que le boîtier d'embout (102) comprend une surface continue et obstrue au moins en partie l'au moins une ouverture d'évent (24, 26) .

11. Le masque de ventilation de la revendication 7, comprenant en outre :
une panoplie de gaz thérapeutique (30) couplée au corps de masque (22), la panoplie de gaz thérapeutique définissant un canal de gaz thérapeutique, la panoplie de gaz thérapeutique comprenant une pluralité d'orifices de gaz thérapeutique (31, 32, 32') en communication fluidique avec le canal de gaz thérapeutique, sachant que la pluralité d'orifices de gaz thérapeutique sont configurés pour créer un flux de gaz thérapeutique à l'intérieur de la cavité de patient.

12. Le masque de ventilation de la revendication 11, sachant que, lorsque l'embout est couplé avec le masque, une partie de l'embout obstrue au moins une partie de la pluralité d'orifices de gaz thérapeutique (31, 32, 32') pour recueillir un gaz sortant de ceux-ci et diriger le gaz recueilli dans le canal de gaz de l'embout.

13. Procédé comprenant :
l'introduction d'un gaz thérapeutique dans une cavité de patient d'un masque de ventilation (20) via une pluralité d'orifices de gaz (31, 32, 32') ;
le fait de diriger un flux de gaz (G) depuis un orifice (31, 32, 32', 41) sur le masque à travers un canal de nébuliseur (124) d'un embout de distribution de médicament (100, 200, 300, 400, 500, 600, 700, 800) couplé avec le masque de ventilation ;
le fait de soutirer une partie d'un médicament (112) depuis un réservoir (110) à travers le canal de nébuliseur (124) avec le flux de gaz, sachant que le réservoir est disposé à l'intérieur de l'embout de distribution de médicament ; et
le fait de diriger le flux de gaz et la partie du médicament dans la cavité de patient du masque de ventilation.

14. Le procédé de la revendication 13, comprenant en outre le fait d'attacher l'embout de distribution de médicament (100, 200, 300, 400, 500, 600, 700, 800, 900) au masque de ventilation (20) .

15. Le procédé de la revendication 14, sachant que le fait d'attacher l'embout de distribution de médicament (100, 200, 300, 400, 500, 600, 700, 800) comprend le fait d'attacher l'embout de distribution de médicament à une ouverture d'évent (24, 26) du masque de ventilation.
